# FASCICULE DE BREVET EUROPEEN

(11) **EP 4 260 776 B1**
(45) Date de publication et mention de la délivrance du brevet: **11.11.2026**
(21) Numéro de dépôt: 23167437.5
(22) Date de dépôt: 11.04.2023
(51) Int. Cl.: A47K 10/48, A45D 20/00, A61L 2/14, A61L 9/22

(54) **DISPOSITIF DE SÉCHAGE, POUR LE SÉCHAGE D'UNE PARTIE DU CORPS D'UN UTILISATEUR**
TROCKNUNGSVORRICHTUNG ZUM TROCKNEN EINES KÖRPERTEILS EINES BENUTZERS
DRYING DEVICE FOR DRYING A BODY PART OF A USER

(30) Priorité: 12.04.2022 FR 2203340
(43) Date de publication de la demande: 18.10.2023
(73) Titulaire: J.V.D. S.A.S., 44400 Rezé (FR)
(72) Inventeur: DE BARBEYRAC, Olivier, 44400 Rezé (FR); RETIERE, Thibault, 44400 Rezé (FR); NIEMIR-DEVEAU, Pierre-Alexandre, 26120 Peyrus (FR); SOLERE, Gaelle, 44400 Rezé (FR)
(74) Mandataire: Jacobacci & Partners France

(56) Documents cités:
- CN-A- 113 558 515
- JP-A- 2013 111 226

## Description

### Domaine technique de l'invention

La présente invention concerne le domaine technique des dispositifs de séchage, pour le séchage d'une partie du corps d'un utilisateur, par exemple sèche-mains ou sèche-cheveux.

### Etat de la technique

De manière classique, les dispositifs de séchage, comme celui divulgué par le document CN 113 558 515 A, comprennent un châssis délimitant un volume interne qui comprend :
- un circuit aéraulique qui est délimité par au moins une entrée et par au moins une sortie, adapté à une circulation d'air depuis ladite au moins une entrée vers ladite au moins une sortie, et
- des moyens ventilateurs, pour la production d'un flux d'air le long dudit circuit aéraulique et pour générer un jet d'air destiné à sécher une partie du corps d'un utilisateur en regard de ladite au moins une sortie.

Certains dispositifs de séchage intègrent des moyens de traitement du flux d'air, par exemple des ensembles d'ionisation qui sont disposés le long du canal d'air et qui émettent des ions chargés directement dans le courant d'air mobile, désinfectant une partie du corps de l'utilisateur.

Toutefois, les dispositifs de séchage actuelles ne sont pas entièrement satisfaisants sur le plan de l'hygiène.

En effet, l'hygiène du circuit aéraulique peut se dégrader avec le temps, avec des répercutions sur la qualité du flux d'air qui en découlent.

En outre, ces dispositifs de séchage n'offrent pas une solution satisfaisante pour participer à l'assainissement de l'air ambiant.

Il existe par conséquent un intérêt de disposer de dispositifs de séchage présentent une hygiène améliorée et pouvant participer à l'assainissement de l'air ambiant.

### Présentation de l'invention

Afin de remédier à l'inconvénient précité de l'état de la technique, la présente invention propose un dispositif de séchage comprenant un châssis délimitant un volume interne qui comprend :
- un circuit aéraulique qui est délimité par au moins une entrée et par au moins une sortie, adapté à une circulation d'air depuis ladite au moins une entrée vers ladite au moins une sortie,
- des moyens ventilateurs, pour la production d'un flux d'air le long dudit circuit aéraulique et adaptés à générer un jet d'air séchant destiné à sécher une partie du corps d'un utilisateur en regard de ladite au moins une sortie,
- des moyens générateur de plasma froid,
- des moyens de commande, pour la commande dudit dispositif de séchage.

Les moyens de commande comprennent deux modules de pilotage:
- un premier module de pilotage, pour le pilotage desdits moyens ventilateurs,
   lequel premier module de pilotage est configuré pour piloter lesdits moyens ventilateurs dans une configuration active de séchage, selon des cycles de séchage successifs, dans laquelle lesdits moyens ventilateurs sont pilotés à une vitesse de séchage adaptée à générer ledit jet d'air séchant, et
- un deuxième module de pilotage, configuré pour piloter lesdits moyens générateur de plasma froid dans une configuration active, selon des cycles de traitement successifs qui sont réguliers et décorrélés desdits cycles de séchage successifs.

De manière générale, au moins un cycle de traitement comprend éventuellement au moins une phase parmi :
- une phase passive, en dehors d'un cycle de séchage, avantageusement afin d'assurer un traitement dudit volume interne, et/ou
- une phase active, lors d'un cycle de séchage, offrant avantageusement une action aéroportée.

Le dispositif de séchage selon l'invention offre ainsi une hygiène améliorée et participe activement à l'assainissement de l'air ambiant.

Les moyens générateur de plasma froid sont ainsi avantageusement autonomes par rapport aux moyens ventilateurs, pour permettre une optimisation de la purification d'air sans être lié à la fonction séchage des mains.

D'autres caractéristiques non limitatives et avantageuses du produit conforme à l'invention, prises individuellement ou selon toutes les combinaisons techniquement possibles, sont les suivantes :
- le deuxième module de pilotage est configuré de sorte que les cycles de traitement successifs sont mis en œuvre de manière régulière, selon au moins une fréquence, voire selon au moins deux fréquences différentes ;
- le premier module de pilotage et le second module de pilotage sont configurés de sorte que le pilotage des moyens ventilateurs est décorrélé par rapport auxdits cycles de traitement successifs ; de manière alternative, le pilotage des moyens ventilateurs est corrélé par rapport auxdits cycles de traitement successifs de sorte que, au cours d'un cycle de traitement et en dehors d'un cycle de séchage, le premier module de pilotage pilote lesdits moyens ventilateurs dans une configuration active de diffusion dans laquelle la vitesse de diffusion, inférieure à ladite vitesse de séchage, est adaptée à générer un jet d'air diffusant destiné à diffuser ledit plasma froid dans l'environnement dudit dispositif de séchage ;
- le deuxième module de pilotage est associé à des moyens de réglage qui sont prévus pour régler la fréquence des cycles de traitement successifs, avantageusement de sorte à tenir compte du volume de l'espace dans lequel est rapporté ledit dispositif de séchage ou de l'intensité d'utilisation du dispositif de séchage équipé ; de préférence, les moyens de réglage consistent en des moyens de temporisation ;
- les moyens ventilateurs comprennent un rotor qui est implanté dans un carter ; et les moyens générateur de plasma froid sont implantés dans le circuit aéraulique, en amont dudit rotor ;
- le volume interne comprend des moyens ventilateurs secondaires, pilotés par ledit deuxième module de pilotage et adaptés à générer un jet d'air diffusant secondaire destiné à diffuser ledit plasma froid dans l'environnement dudit dispositif de séchage ; et le deuxième module de pilotage est configuré de sorte que, au cours d'un cycle de traitement, lesdits moyens ventilateurs secondaires sont pilotés dans une configuration active de diffusion adaptée à générer un jet d'air diffusant secondaire destiné à diffuser ledit plasma froid dans l'environnement dudit dispositif de séchage.

La présente invention concerne encore le procédé de fonctionnement d'un dispositif de séchage selon l'invention.

Les moyens ventilateurs sont pilotés dans une configuration active selon des cycles de séchage successifs.

Et les moyens générateur de plasma froid sont pilotés dans une configuration active selon des cycles de traitement successifs, réguliers et décorrélés desdits cycles de séchage successifs.

D'autres caractéristiques non limitatives et avantageuses du procédé conforme à l'invention, prises individuellement ou selon toutes les combinaisons techniquement possibles, sont les suivantes :
- les cycles de traitement successifs sont mis en œuvre de manière régulière, selon au moins une fréquence, voire selon au moins deux fréquences différentes (par exemple une fréquence élevée et une fréquence réduite) ;
- le pilotage des moyens ventilateurs est décorrélé desdits cycles de traitement successifs ; ou le pilotage des moyens ventilateurs est corrélé par rapport auxdits cycles de traitement successifs de sorte que, au cours d'un cycle de traitement et en dehors d'un cycle de séchage, lesdits moyens ventilateurs sont pilotés à une vitesse de diffusion, inférieure à ladite vitesse de séchage, adaptée à générer un jet d'air diffusant destiné à diffuser ledit plasma froid dans l'environnement dudit dispositif de séchage.

Dans le cas de moyens ventilateurs décorrélés des cycles de traitement successifs, chaque cycle de traitement comprend avantageusement au moins une phase parmi :
- une phase passive, en dehors dudit cycle de séchage, avantageusement afin d'assurer un traitement dudit volume interne, et/ou
- une phase active, lors d'un cycle de séchage, offrant avantageusement une action aéroportée.

Bien entendu, les différentes caractéristiques, variantes et formes de réalisation de l'invention peuvent être associées les unes avec les autres selon diverses combinaisons dans la mesure où elles ne sont pas incompatibles ou exclusives les unes des autres.

### Description détaillée de l'invention

De plus, diverses autres caractéristiques de l'invention ressortent de la description annexée effectuée en référence aux dessins qui illustrent des formes, non limitatives, de réalisation de l'invention et où :
[Fig. 1] est une vue schématique d'un dispositif de séchage selon un premier mode de réalisation, avec une coupe longitudinale pour illustrer son volume interne, au cours d'une phase passive d'un cycle de traitement (en dehors d'un cycle de séchage) ;
[Fig. 2] est encore une vue schématique du dispositif de séchage selon la figure 1, au cours d'une phase active d'un cycle de traitement (lors d'un cycle de séchage) ;
[Fig. 3] est une vue schématique d'un dispositif de séchage selon un second mode de réalisation, avec une coupe longitudinale pour illustrer son volume interne.

Il est à noter que, sur ces figures, les éléments structurels et/ou fonctionnels communs aux différentes variantes peuvent présenter les mêmes références.

La présente invention concerne ainsi un dispositif de séchage 1, par exemple un sèche-mains ou un sèche-cheveux, consistant en un dispositif qui est apte à sécher une partie du corps d'un utilisateur (par exemple les mains ou les cheveux) par un flux d'air et qui fonctionne à l'énergie électrique.

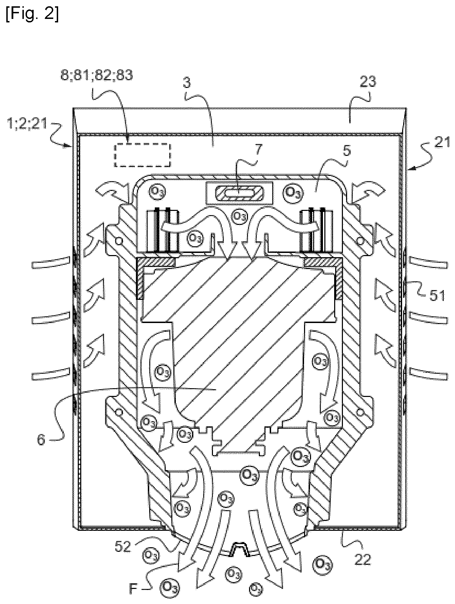

De manière générale, ce dispositif de séchage 1 comprend un châssis 2 délimitant un volume interne 3 qui comprend une combinaison de caractéristiques techniques, à savoir :
- un circuit aéraulique 5, adapté à une circulation d'air,
- des moyens ventilateurs 6, pour la production d'un flux d'air le long du circuit aéraulique 5 et pour générer un jet d'air séchant F destiné à sécher une partie du corps d'un utilisateur (figure 2),
- des moyens générateur de plasma froid 7, et
- des moyens de commande 8, pour la commande de ce dispositif de séchage 1.

### Châssis

Le châssis 2 est formé avantageusement par un carter ou un boîtier, de forme globalement parallélépipédique, par exemple réalisé en matériau plastique et/ou métallique.

Ce châssis 2 comprend avantageusement un ensemble de faces :
- une face arrière (non représentée), destinée à venir en appui et être fixée sur un support vertical (par exemple un mur),
- une face avant (non représentée), en regard de laquelle vient se placer l'utilisateur,
- deux faces latérales 21,
- une face inférieure 22, destinée à être orientée vers le sol, et
- une face supérieure 23, destinée à être orientée vers le plafond.

Le dispositif de séchage 1 consiste avantageusement en un dispositif de séchage mural, avantageusement fixé en applique sur un mur de bâtiment.

### Circuit aéraulique

Le circuit aéraulique 5 chemine dans le volume interne 3 et il est délimité par au moins une entrée 51 et par au moins une sortie 52.

Ladite au moins une entrée 51 et ladite au moins une sortie 52, ménagées avantageusement dans les faces du châssis 2 et formant avantageusement un conduit de ventilation.

En l'espèce, ladite au moins une entrée 51 est ici ménagée dans les faces latérales 21 ; et ladite au moins une sortie 52 est ici ménagée dans la face inférieure 22.

Ce circuit aéraulique 5 est adapté à une circulation d'air depuis ladite au moins une entrée 51 vers ladite au moins une sortie 52, sous l'action des moyens ventilateurs 6.

### Moyens ventilateurs

Les moyens ventilateurs 6 sont adaptés à la production d'un flux d'air le long du circuit aéraulique 5, depuis ladite au moins une entrée 51 vers ladite au moins une sortie 52.

Ces moyens ventilateurs 6 sont en outre adaptés à générer un jet d'air séchant F destiné à sécher une partie du corps d'un utilisateur en regard de ladite au moins une sortie 52.

Les moyens ventilateurs 6 consistent avantageusement en un ventilateur électrique, classique en soi.

De préférence, ces moyens ventilateurs 6 comprennent un rotor 61 qui est implanté dans un carter 62, formant un bloc (ou module) ventilateur.

Ces moyens ventilateurs 6 sont avantageusement implantés sur le circuit aéraulique 5, entre ladite au moins une entrée 51 et ladite au moins une sortie 52.

En d'autres termes, ces moyens ventilateurs 6 forment avantageusement un tronçon du circuit aéraulique 5.

### Moyens générateur de plasma froid

Les moyens générateur de plasma froid 7 sont avantageusement implantés dans le circuit aéraulique 5.

De préférence, les moyens générateur de plasma froid 7 sont implantés en amont des moyens ventilateurs 6, entre ladite au moins une entrée 51 et lesdits moyens ventilateurs 6.

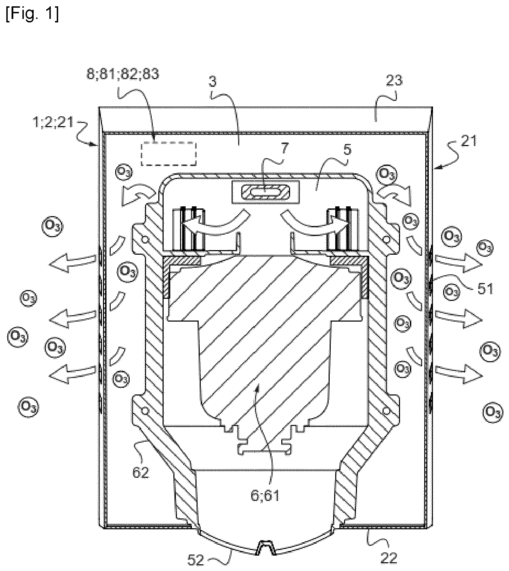

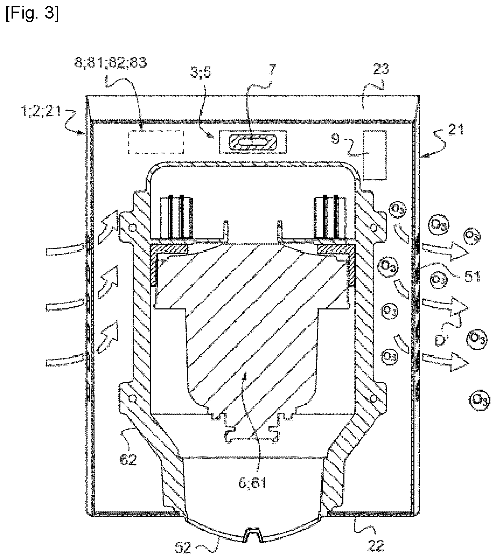

En l'espèce, les moyens générateur de plasma froid 7 sont ici implantés en amont du rotor 61, avantageusement :
- au sein du carter 62 des moyens ventilateurs 6 (selon le mode de réalisation illustré sur la figure 1), ou
- à l'extérieur du carter 62 des moyens ventilateurs 6, de préférence en amont (selon le mode de réalisation illustré sur la figure 3).

Les moyens générateur de plasma froid 7 vont ainsi permettre de traiter et de purifier les surfaces intérieures du dispositif de séchage 1, avantageusement le volume interne 3 et en particulier le long du circuit aéraulique 5, garantissant une bonne qualité de l'air soufflé sur l'utilisateur.

Les moyens générateur de plasma froid 7 participent également à l'assainissement de l'environnement dans lequel le dispositif de séchage 1 est implanté, comme développé par la suite en lien avec les moyens de commande 8.

Par « plasma froid », on entend en particulier des particules ionisées qui se forment autour de décharges électriques (étincelles).

Pour cela, les moyens générateurs de plasma froid 7 consistent par exemple en un transformateur piézoélectrique pour la génération de plasma froid directement à leur surface (Piezoelectric Direct Discharge Plasma ou PDDP, ou décharges à barrière diélectrique ou DBD) sur une plaque SiO₂ et/ou une plaque semi-conductrice.

De tels moyens générateurs de plasma froid 7 présente avantageusement au moins un effet parmi un effet ozonant, un effet ionisant et un effet polarisant.

### Moyens de commande

Les moyens de commande 8 assurent le pilotage des moyens ventilateurs 6 et des moyens générateur de plasma froid 7.

Ces moyens de commande 8 consistent par exemple en au moins une carte électronique qui comprend un circuit imprimé comportant les composants électroniques et/ou électriques utiles au fonctionnement de ce dispositif de séchage 1 (par exemple un microcontrôleur associé à un programme d'ordinateur).

En l'espèce, les moyens de commande 8 comprennent deux modules de pilotage 81, 82 :
- un premier module de pilotage 81, pour le pilotage des moyens ventilateurs 6 au moins dans une configuration active de séchage selon des cycles de séchage successifs, et
- un deuxième module de pilotage 82, pour le pilotage des moyens générateur de plasma froid 7 dans une configuration active selon des cycles de traitement successifs.

Les deux modules de pilotage 81, 82 sont avantageusement indépendants, avec des réglages respectifs.

Le premier module de pilotage 81 est avantageusement apte à piloter les moyens ventilateurs 6 entre au moins deux configurations :
- une configuration active de séchage, dans laquelle les moyens ventilateurs 6 (en particulier le rotor 61) sont pilotés à une vitesse de séchage adaptée à générer un flux d'air au sein du circuit aéraulique 5 et en corolaire le jet d'air séchant F au travers de ladite au moins une sortie 52,
- une configuration inactive (le rotor 61 étant à l'arrêt), dans laquelle les ventilateurs 6 ne génèrent pas ledit flux d'air ni ledit jet d'air séchant F (rotor 61 étant à l'arrêt), et éventuellement
- une configuration active de diffusion dans laquelle lesdits moyens ventilateurs 6 (en particulier le rotor 61) sont pilotés à une vitesse de diffusion, inférieure à ladite vitesse de séchage, adaptée à générer un jet d'air diffusant D destiné à diffuser le plasma froid dans l'environnement dudit dispositif de séchage 1.

Par « cycle de séchage », on entend avantageusement une période au cours de laquelle le premier module de pilotage 81 maintient les moyens ventilateurs 6 en configuration active dans la vitesse de séchage.

Par exemple, un cycle de séchage correspond à une période variable, initiée après détection de la présence des mains de l'utilisateur et s'arrêtant après éloignement des mains de l'utilisateur.

Pour cela, les moyens ventilateurs 6 coopèrent avantageusement, de manière classique en soi, avec des moyens de détection de présence (non représentés), pour la détection de la présence d'une partie du corps de l'utilisateur (mains ou tête par exemple) dans une zone de séchage qui s'étend en regard de ladite au moins une sortie 52.

De tels moyens de détection de présence équipent avantageusement le dispositif de séchage 1.

Les cycles de séchage successifs sont ainsi liés à l'utilisation du dispositif de séchage 1 par les utilisateurs.

Le deuxième module de pilotage 82 est avantageusement apte à piloter les moyens générateur de plasma froid 7 dans une configuration active selon des cycles de traitement successifs.

Le deuxième module de pilotage 82 est alors avantageusement apte à piloter les moyens générateur de plasma froid 7 entre deux configurations :
- une configuration active (en marche), dans laquelle les moyens générateur de plasma froid 7 génèrent des particules ionisées, et
- une configuration inactive (à l'arrêt), dans laquelle les moyens générateur de plasma froid 7 ne génèrent pas de particules ionisées.

Par « cycle de traitement », on entend avantageusement une période au cours de laquelle le deuxième module de pilotage 82 maintient les moyens générateur de plasma froid 7 dans la configuration active.

Selon l'invention, le deuxième module de pilotage 82 est configuré pour piloter lesdits moyens générateur de plasma froid 7 selon des cycles de traitement successifs qui sont réguliers et décorrélés (ou indépendants) des cycles de séchage successifs.

Par « décorrélés », on entend avantageusement des cycles de traitement et des cycles de séchage qui ne sont pas synchrones.

Un cycle de traitement et un cycle de séchage se produisent alors à des moments indépendants l'un de l'autre.

En d'autres termes, les cycles de traitement sont avantageusement indépendants des cycles de séchages et déclenchés automatiquement, de sorte qu'un cycle de traitement peut se produire lors d'un cycle de séchage et/ou en dehors d'un cycle de séchage.

Encore en d'autres termes, le déclanchement des cycles de traitement est indépendant du déclanchement des cycles de séchage.

Le deuxième module de pilotage 82 est avantageusement configuré de sorte que les cycles de traitement successifs sont mis en œuvre de manière régulière.

Par « régulière », on entend avantageusement un cycle de traitement dont la durée est déterminée, avec un intervalle de temps également déterminé entre deux cycles de traitement.

Le deuxième module de pilotage 82 est encore avantageusement configuré de sorte que les cycles de traitement successifs sont mis en œuvre de manière régulière, selon au moins une fréquence, voire selon au moins deux fréquences différentes avantageusement réparties sur une période quotidienne.

Par « fréquence », on attend la durée du cycle de traitement et/ou l'intervalle de temps entre deux cycles de traitement.

Par exemple, le deuxième module de pilotage 82 est configuré de sorte à mettre en œuvre au moins deux fréquences, par exemple une fréquence élevée et une fréquence réduite (voire également une fréquence intermédiaire).

Une fréquence « élevée » correspond à une durée du cycle de traitement supérieure, et/ou un intervalle de temps réduit entre deux cycles de traitement, par rapport à une fréquence « réduite ».

Les fréquences différentes sont avantageusement mises en œuvre pour tenir compte de l'intensité d'usage du dispositif de séchage 1.

Par exemple, une fréquence « élevée » est sélectionnée pour les heures d'accès au dispositif de séchage 1 ; et une fréquence « réduite » est sélectionnée pour les heures de restriction d'accès au dispositif de séchage 1.

De préférence encore, la durée du cycle de traitement est fixe. Et un intervalle de temps entre deux cycles de traitement successifs correspond à 1 à 10 fois, la durée du cycle de traitement.

A cet égard, la durée d'un cycle de traitement est avantageusement de 20 secondes à 7 200 secondes. Et deux cycles de traitement sont avantageusement séparés d'une durée allant de 1 à 10 fois la durée du cycle de traitement.

Par exemple et sans être limitatif, la durée d'un cycle de traitement est de 30 s à 90 s ; deux cycles de traitement sont avantageusement séparés d'une durée allant de 1 min à 10 min.

De tels cycles de traitement réguliers permettent un traitement continu de l'environnement, y compris lorsque les moyens ventilateurs 6 restent dans une configuration inactive.

De préférence encore, le deuxième module de pilotage 82 est associé à des moyens de réglage 83 qui sont prévus pour régler ladite au moins une fréquence des cycles de traitement successifs.

Cette fonctionnalité permet avantageusement de prendre en compte le volume de l'espace (avantageusement une pièce d'un bâtiment, par exemple un sanitaire) dans lequel est rapporté le dispositif de séchage 1. Elle permet également de prendre en compte la régularité d'usage de ce dispositif de séchage 1 ou encore du passage dans l'espace équipé.

En d'autres termes, la fréquence est avantageusement augmentée avec le volume de l'espace dans lequel est rapporté le dispositif de séchage 1 et/ou avec la régularité d'usage et/ou du passage dans l'espace équipé, et inversement.

Pour cela, les moyens de réglage 83 consistent avantageusement en des moyens de temporisation, avantageusement sous la forme d'un potentiomètre.

Un opérateur peut intervenir sur ces moyens de réglage 83 de sorte à régler, à façon, la fréquence (durée et/ou intervalle par exemple) des cycles de traitement successifs.

Selon un premier mode de réalisation, les moyens générateur de plasma froid 7 peuvent être autonomes par rapport aux moyens ventilateurs 6, notamment pour permettre une purification d'air sans être lié à la fonction séchage des mains.

Le pilotage des moyens ventilateurs 6 est ainsi décorrélé par rapport aux cycles de traitement successifs.

Les cycles de traitement sont ainsi avantageusement cycliques, avec une configuration active et une configuration inactive dont les durées respectives peuvent être ajustées à façon.

Un cycle de traitement comprend alors avantageusement au moins une phase, voire deux phases, parmi :
- une phase passive, en dehors d'un cycle de séchage, de préférence afin d'assurer un traitement du volume interne 3, et/ou
- une phase active, lors d'un cycle de séchage, de préférence offrant une action aéroportée.

Lors de la phase passive (figure 1), les particules ionisées vont avoir tendance à diffuser au sein du circuit aéraulique 5, ici principalement vers l'amont (vers ladite au moins une entrée 51).

Cette phase passive est intéressante pour assurer un traitement du circuit aéraulique 5, y compris sur un tronçon amont par rapport aux moyens générateur de plasma froid 7.

Cette phase passive permet d'éviter un développement de toutes contaminations internes liées à l'utilisation et à l'environnement.

Les particules ionisées se répartissent avantageusement naturellement, par diffusion.

Lors de la phase active (figure 2), le flux d'air entraîne les particules ionisées vers l'aval par rapport aux moyens générateur de plasma froid 7 (vers ladite au moins une sortie 52).

Cette phase active est intéressante pour assurer un traitement du circuit aéraulique 5, sur un tronçon aval par rapport aux moyens générateur de plasma froid 7.

Elle permet également l'action aéroportée, c'est-à-dire favoriser une diffusion des particules ionisées au niveau du jet d'air séchant F destiné à sécher une partie du corps d'un utilisateur (tout en prévenant une contamination de l'utilisateur par ce jet d'air séchant F).

De manière alternative, selon un second mode de réalisation, les moyens générateur de plasma froid 7 peuvent intervenir sur le fonctionnement des moyens ventilateurs 6, notamment pour favoriser une purification de l'air ambiant.

Le pilotage des moyens ventilateurs 6 peuvent alors être corrélé par rapport aux cycles de traitement successifs.

A cet égard, au cours d'un cycle de traitement et en dehors d'un cycle de séchage, le premier module de pilotage 81 pilote avantageusement les moyens ventilateurs 6 dans une configuration active de diffusion dans laquelle ces moyens ventilateurs 6 sont pilotés à la vitesse de diffusion, inférieure à la vitesse de séchage.

Cette vitesse de diffusion est alors adaptée à générer un jet d'air diffusant D destiné à diffuser le plasma froid dans l'environnement dudit dispositif de séchage 1.

Par exemple, cette vitesse de diffusion est réduite de 30 à 90 % par rapport à la vitesse de séchage.

De manière générale, les moyens de commande 8 peuvent être connectés à un serveur distant, via des moyens de télécommunication, afin d'assurer des fonctionnalités supplémentaires (relevé de fréquentation, gestion des interventions prédictives, etc.) et des possibilités de pilotage à distance.

### Ventilateur secondaire

Selon un mode de réalisation représenté sur la figure 3, le volume interne 3 comprend des moyens ventilateurs secondaires 9, pilotés par le deuxième module de pilotage 82.

Le volume interne 3 comprend ainsi :
- les moyens ventilateurs 6 précités, adaptés à générer le jet d'air séchant F, formant alors des moyens ventilateurs 6 principaux, et
- les moyens ventilateurs secondaires 9.

Les moyens ventilateurs secondaires 9 sont avantageusement implantés dans le circuit aéraulique 5, en amont des moyens ventilateurs 6 précités.

Les moyens ventilateurs secondaires 9 sont en communication aéraulique avec les moyens générateur de plasma froid 7.

Ces moyens ventilateurs secondaires 9 sont adaptés à générer un jet d'air diffusant secondaire D', destiné à diffuser le plasma froid dans l'environnement du dispositif de séchage 1.

Pour cela, le deuxième module de pilotage 82 est configuré de sorte que, au cours d'un cycle de traitement, les moyens ventilateurs secondaires 9 sont pilotés dans une configuration active de diffusion adaptée à générer un jet d'air diffusant secondaire D' destiné à diffuser le plasma froid dans l'environnement du dispositif de séchage 1.

Les moyens ventilateurs secondaires 9 sont avantageusement inactifs ou stoppés lorsque les moyens ventilateurs 6 principaux sont en état actif.

Ce jet d'air diffusant secondaire D' chemine avantageusement au travers des ouvertures du circuit aéraulique 5, par exemple au travers de ladite au moins une entrée 51.

### Procédé de fonctionnement

Le procédé de fonctionnement du dispositif de séchage 1 selon l'invention est encore décrit plus en détails ci-dessous en relation avec les figures 1 à 3.

En pratique, les moyens ventilateurs 6 sont pilotés dans une configuration active de séchage selon des cycles de séchage successifs.

Un cycle de séchage est avantageusement initié / déclenché après détection de la présence des mains de l'utilisateur.

Les moyens générateur de plasma froid 7 sont quant à eux pilotés dans une configuration active selon des cycles de traitement successifs qui sont mis en œuvre de manière régulière et décorrélés desdits cycles de séchage successifs.

Les cycles de traitement successifs et les cycles de séchage successifs sont ainsi avantageusement décorrélés.

Selon un premier mode de fonctionnement, le pilotage des moyens ventilateurs 6 est décorrélé des cycles de traitement successifs.

Chaque cycle de traitement comprend ainsi au moins une phase parmi :
- une phase passive, en dehors d'un cycle de séchage, afin d'assurer un traitement dudit volume interne 3, et/ou
- une phase active, lors d'un cycle de séchage, offrant une action aéroportée.

Selon un deuxième mode de fonctionnement, le pilotage des moyens ventilateurs 6 est corrélé avec les cycles de traitement successifs.

A cet égard, au cours d'un cycle de traitement et en dehors d'un cycle de séchage, les moyens ventilateurs 6 sont pilotés dans la configuration active de diffusion dans laquelle les moyens ventilateurs 6 sont pilotés dans la vitesse de diffusion (inférieure à la vitesse de séchage).

Le jet d'air diffusant D est alors destiné à diffuser le plasma froid dans l'environnement du dispositif de séchage 1.

Au cours d'un cycle de traitement, un éventuel cycle de séchage génère une commutation temporaire des moyens ventilateurs 6 dans la configuration active de séchage (selon la vitesse de séchage précité).

Par ailleurs, en présence de moyens ventilateurs secondaires 9, un jet d'air diffusant secondaire D' est avantageusement généré pendant les cycles de traitement de sorte à diffuser le plasma froid dans l'environnement du dispositif de séchage 1.

Bien entendu, diverses autres modifications peuvent être apportées à l'invention dans le cadre des revendications annexées.

## Revendications

1. Dispositif de séchage, pour le séchage d'une partie du corps d'un utilisateur, comprenant un châssis (2) délimitant un volume interne (3) qui comprend :
- un circuit aéraulique (5) qui est délimité par au moins une entrée (51) et par au moins une sortie (52), adapté à une circulation d'air depuis ladite au moins une entrée (51) vers ladite au moins une sortie (52),
- des moyens ventilateurs (6), pour la production d'un flux d'air le long dudit circuit aéraulique (5) et adaptés à générer un jet d'air séchant (F) destiné à sécher une partie du corps d'un utilisateur en regard de ladite au moins une sortie (52),
- des moyens générateur de plasma froid (7), et
- des moyens de commande (8), pour la commande dudit dispositif de séchage (1), lesquels moyens de commande (8) comprennent deux modules de pilotage (81, 82) :
- un premier module de pilotage (81), pour le pilotage desdits moyens ventilateurs (6),
lequel premier module de pilotage (81) est configuré pour piloter lesdits moyens ventilateurs (6) dans une configuration active de séchage, selon des cycles de séchage successifs, dans laquelle lesdits moyens ventilateurs (6) sont pilotés à une vitesse de séchage adaptée à générer ledit jet d'air séchant (F), et
- un deuxième module de pilotage (82), configuré pour piloter lesdits moyens générateur de plasma froid (7) dans une configuration active, selon des cycles de traitement successifs qui sont réguliers et décorrélés desdits cycles de séchage successifs.

2. Dispositif de séchage, selon la revendication 1, **caractérisé en ce que** le deuxième module de pilotage (82) est configuré de sorte que les cycles de traitement successifs sont mis en œuvre de manière régulière, selon au moins une fréquence.

3. Dispositif de séchage, selon la revendication 2, **caractérisé en ce que** le deuxième module de pilotage (82) est configuré de sorte que les cycles de traitement successifs sont mis en œuvre de manière régulière, selon au moins deux fréquences différentes.

4. Dispositif de séchage, selon l'une quelconque des revendications 1 à 3, **caractérisé en ce que** le premier module de pilotage (81) et le second module de pilotage (82) sont configurés de sorte que :
- le pilotage des moyens ventilateurs (6) est décorrélé par rapport auxdits cycles de traitement successifs, ou
- le pilotage des moyens ventilateurs (6) est corrélé par rapport auxdits cycles de traitement successifs de sorte que, au cours d'un cycle de traitement et en dehors d'un cycle de séchage, le premier module de pilotage (81) pilote lesdits moyens ventilateurs (6) dans une configuration active de diffusion dans laquelle une vitesse de diffusion, inférieure à ladite vitesse de séchage, est adaptée à générer un jet d'air diffusant D destiné à diffuser ledit plasma froid dans l'environnement dudit dispositif de séchage (1).

5. Dispositif de séchage, selon l'une quelconque des revendications 1 à 4, **caractérisé en ce que** le deuxième module de pilotage (82) est associé à des moyens de réglage (83) qui sont prévus pour régler la fréquence des cycles de traitement successifs.

6. Dispositif de séchage, selon la revendication 5, **caractérisé en ce que** les moyens de réglage (83) consistent en des moyens de temporisation.

7. Dispositif de séchage, selon l'une quelconque des revendications 1 à 6, **caractérisé en ce que** les moyens ventilateurs (6) comprennent un rotor (61) qui est implanté dans un carter (62),
et **en ce que** les moyens générateur de plasma froid (7) sont implantés dans le circuit aéraulique (5), en amont dudit rotor (61).

8. Dispositif de séchage, selon l'une quelconque des revendications 1 à 7, **caractérisé en ce que** le volume interne (3) comprend des moyens ventilateurs secondaires (9), pilotés par ledit deuxième module de pilotage (82) et adaptés à générer un jet d'air diffusant secondaire (D') destiné à diffuser ledit plasma froid dans l'environnement dudit dispositif de séchage (1),
et que le deuxième module de pilotage (82) est configuré de sorte que, au cours d'un cycle de traitement, lesdits moyens ventilateurs secondaires (9) sont pilotés dans une configuration active de diffusion adaptée à générer un jet d'air diffusant secondaire (D') destiné à diffuser ledit plasma froid dans l'environnement dudit dispositif de séchage (1).

9. Procédé de fonctionnement d'un dispositif de séchage (1) selon l'une quelconque des revendications 1 à 8, **caractérisé en ce que** les moyens ventilateurs (6) sont pilotés dans une configuration active selon des cycles de séchage successifs,
**en ce que** les moyens générateur de plasma froid (7) sont pilotés dans une configuration active selon des cycles de traitement successifs, réguliers et décorrélés desdits cycles de séchage successifs.

10. Procédé de fonctionnement d'un dispositif de séchage (1) selon la revendication 9, **caractérisé en ce que** les cycles de traitement successifs sont mis en œuvre de manière régulière, selon au moins une fréquence, voire selon au moins deux fréquences différentes.

11. Procédé de fonctionnement d'un dispositif de séchage (1) selon l'une quelconque des revendications 9 ou 10, **caractérisé en ce que** le pilotage des moyens ventilateurs (6) est décorrélé desdits cycles de traitement successifs.

12. Procédé de fonctionnement d'un dispositif de séchage (1) selon l'une quelconque des revendications 9 ou 10, **caractérisé en ce que** le pilotage des moyens ventilateurs (6) est corrélé par rapport auxdits cycles de traitement successifs de sorte que, au cours d'un cycle de traitement et en dehors d'un cycle de séchage, lesdits moyens ventilateurs (6) sont pilotés à une vitesse de diffusion, inférieure à ladite vitesse de séchage, adaptée à générer un jet d'air diffusant (D) destiné à diffuser ledit plasma froid dans l'environnement dudit dispositif de séchage (1).

## Patentansprüche

1. Trocknungsvorrichtung zum Trocknen eines Körperteils eines Benutzers mit einem Gestell (2), das ein inneres Volumen (3) abgrenzt, das
- einen Lüftungskreis (5), der durch mindestens einen Eingang (51) und mindestens einen Ausgang (52) abgegrenzt ist und für einen Luftstrom von dem mindestens einen Eingang (51) zum mindestens einen Ausgang (52) ausgelegt ist,
- Lüftungsmittel (6) zum Erzeugen eines Luftstroms entlang des Lüftungskreises (5), die dazu ausgelegt sind, einen trocknenden Luftstrahl (F) zu erzeugen, der dazu bestimmt ist, einen dem mindestens einen Ausgang (52) gegenüberliegenden Teil des Körpers eines Benutzers zu trocknen,
- Mittel (7) zum Erzeugen eines kalten Plasmas und
- Steuerungsmittel (8) zum Steuern der Trocknungsvorrichtung (1) aufweist,
wobei die Steuerungsmittel (8) zwei Steuerungsmodule (81, 82) aufweisen:
- ein erstes Steuerungsmodul (81) zum Steuern der Lüftungsmittel (6),
wobei das erste Steuerungsmodul (81) dazu ausgelegt ist, die Lüftungsmittel (6) in aufeinanderfolgenden Trocknungszyklen in einer aktiven Trocknungskonfiguration zu steuern, in der die Lüftungsmittel (6) bei einer Trocknungsgeschwindigkeit gesteuert werden, die geeignet ist, einen trocknenden Luftstrahl (F) zu erzeugen, und
- ein zweites Steuerungsmodul (82), das dazu ausgelegt ist, die Mittel (7) zum Erzeugen eines kalten Plasmas in einer aktiven Konfiguration in aufeinanderfolgenden Behandlungszyklen zu steuern, die regelmäßig und von den aufeinanderfolgenden Trocknungszyklen unabhängig sind.

2. Trocknungsvorrichtung gemäß Anspruch 1, **dadurch gekennzeichnet, daß** das zweite Steuerungsmodul (82) so ausgelegt ist, daß die aufeinanderfolgenden Behandlungszyklen regelmäßig mit mindestens einer Frequenz durchgeführt werden.

3. Trocknungsvorrichtung gemäß Anspruch 2, **dadurch gekennzeichnet, daß** das zweite Steuerungsmodul (82) so ausgelegt ist, daß die aufeinanderfolgenden Behandlungszyklen regelmäßig mit mindestens zwei verschiedenen Frequenzen durchgeführt werden.

4. Trocknungsvorrichtung gemäß einem der Ansprüche 1 bis 3, **dadurch gekennzeichnet, daß** das erste Steuerungsmodul (81) und das zweite Steuerungsmodul (82) so ausgelegt sind, daß:
- die Steuerung der Lüftungsmittel (6) von den aufeinanderfolgenden Behandlungszyklen unabhängig ist, oder
- die Steuerung der Lüftungsmittel (6) mit den aufeinanderfolgenden Behandlungszyklen so korreliert ist, daß das erste Steuerungsmodul (81) während eines Behandlungszyklus und außerhalb eines Trocknungszyklus die Lüftungsmittel (6) in einer aktiven Ausstrahlungskonfiguration steuert, in der eine Ausstrahlungsgeschwindigkeit, die geringer als die Trocknungsgeschwindigkeit ist, geeignet ist, einen ausstrahlenden Luftstrahl (D) zu erzeugen, der dazu bestimmt ist, das kalte Plasma in der Umgebung der Trocknungsvorrichtung (1) zu verteilen.

5. Trocknungsvorrichtung gemäß einem der Ansprüche 1 bis 4, **dadurch gekennzeichnet, daß** das zweite Steuerungsmodul (82) Regelungsmitteln (83) zugeordnet ist, die vorgesehen sind, um die Frequenz der aufeinanderfolgenden Behandlungszyklen zu regeln.

6. Trocknungsvorrichtung gemäß Anspruch 5, **dadurch gekennzeichnet, daß** die Regelungsmittel (83) aus Verzögerungsmitteln bestehen.

7. Trocknungsvorrichtung gemäß einem der Ansprüche 1 bis 6, **dadurch gekennzeichnet, daß** die Lüftungsmittel (6) einen Rotor (61) aufweisen, der in einem Gehäuse (62) eingesetzt ist,
und daß die Mittel (7) zum Erzeugen eines kalten Plasmas im Lüftungskreis (5) stromaufwärts vom Rotor (61) eingesetzt sind.

8. Trocknungsvorrichtung gemäß einem der Ansprüche 1 bis 7, **dadurch gekennzeichnet, daß** das innere Volumen (3) sekundäre Lüftungsmittel (9) aufweist, die vom zweiten Steuerungsmodul (82) gesteuert werden und dazu ausgelegt sind, einen sekundären ausstrahlenden Luftstrahl (D') zu erzeugen, der dazu bestimmt ist, das kalte Plasma in der Umgebung der Trocknungsvorrichtung (1) zu verteilen, und daß das zweite Steuerungsmodul (82) so ausgelegt ist, daß die sekundären Lüftungsmittel (9) während eines Behandlungszyklus in einer aktiven Ausstrahlungskonfiguration gesteuert werden, die dazu ausgelegt ist, einen sekundären ausstrahlenden Luftstrahl (D') zu erzeugen, der dazu bestimmt ist, das kalte Plasma in der Umgebung der Trocknungsvorrichtung (1) zu verteilen.

9. Verfahren zum Betreiben einer Trocknungsvorrichtung (1) gemäß einem der Ansprüche 1 bis 8, **dadurch gekennzeichnet, daß** die Lüftungsmittel (6) in einer aktiven Konfiguration mit aufeinanderfolgenden Trocknungszyklen gesteuert werden
und daß die Mittel (7) zum Erzeugen von Plasma in einer aktiven Konfiguration mit regelmäßigen, von den aufeinanderfolgenden Trocknungszyklen unabhängigen aufeinanderfolgenden Behandlungszyklen gesteuert werden.

10. Verfahren zum Betreiben einer Trocknungsvorrichtung (1) gemäß Anspruch 9, **dadurch gekennzeichnet, daß** die aufeinanderfolgenden Behandlungszyklen regelmäßig mit mindestens einer Frequenz oder mit mindestens zwei verschiedenen Frequenzen durchgeführt werden.

11. Verfahren zum Betreiben einer Trocknungsvorrichtung (1) gemäß einem der Ansprüche 9 oder 10, **dadurch gekennzeichnet, daß** die Steuerung der Lüftungsmittel (6) von den aufeinanderfolgenden Behandlungszyklen unabhängig ist.

12. Verfahren zum Betreiben einer Trocknungsvorrichtung (1) gemäß einem der Ansprüche 9 oder 10, **dadurch gekennzeichnet, daß** die Steuerung der Lüftungsmittel (6) mit den aufeinanderfolgenden Behandlungszyklen so korreliert ist, daß die Lüftungsmittel (6) während eines Behandlungszyklus und außerhalb eines Trocknungszyklus mit einer Ausstrahlungsgeschwindigkeit gesteuert werden, die geringer als die Trocknungsgeschwindigkeit ist und geeignet ist, einen ausstrahlenden Luftstrahl (D) zu erzeugen, der dazu bestimmt ist, das kalte Plasma in der Umgebung der Trocknungsvorrichtung (1) zu verteilen.

## Claims

1. Drying device for drying a part of a user's body, comprising a frame (2) that defines an inner volume (3) which comprises:
- a ventilation circuit (5) which is delimited by at least one inlet (51) and at least one outlet (52) and is designed for an airflow from the at least one inlet (51) to the at least one outlet (52),
- ventilation means (6) for generating an airflow along the ventilation circuit (5) designed to produce a drying air jet (F) intended to dry a part of a user's body in front of the at least one outlet (52),
- means (7) for generating a cold plasma and
- control means (8) for controlling the drying device (1),
wherein the control means (8) comprise two control modules (81, 82):
- a first control module (81) for controlling the ventilation means (6),
wherein the first control module (81) is designed to control the ventilation means (6) in successive drying cycles in an active drying configuration in which the ventilation means (6) are controlled at a drying velocity suitable for generating a drying air jet (F), and
- a second control module (82) designed to control the means (7) for generating a cold plasma in an active configuration in successive treatment cycles that are regular and independent of the successive drying cycles.

2. Drying device according to claim 1, **characterized in that** the second control module (82) is designed such that the successive treatment cycles are carried out regularly at at least one rate.

3. Drying device according to claim 2, **characterized in that** the second control module (82) is designed such that the successive treatment cycles are regularly carried out at at least two different rates.

4. Drying device according to one of claims 1 to 3, **characterized in that** the first control module (81) and the second control module (82) are designed such that:
- the control of the ventilation means (6) is independent of the successive treatment cycles, or
- the control of the ventilation means (6) is correlated with the successive treatment cycles such that the first control module (81) controls the ventilation means (6) during a treatment cycle and outside a drying cycle in an active diffusion configuration, in which a diffusion velocity lower than the drying velocity is suitable to generate a diffusing air jet (D) intended to distribute the cold plasma in the vicinity of the drying device (1).

5. Drying device according to one of claims 1 to 4, **characterized in that** the second control module (82) is associated with adjustment means (83) which are provided to adjust the rate of the successive treatment cycles.

6. Drying device according to claim 5, **characterized in that** the adjustment means (83) consist of delay means.

7. Drying device according to one of claims 1 to 6, **characterized in that** the ventilation means (6) have a rotor (61) which is inserted in a housing (62),
and **in that** the means (7) for generating a cold plasma are inserted in the ventilation circuit (5) upstream of the rotor (61).

8. Drying device according to one of claims 1 to 7, **characterized in that** the inner volume (3) has secondary ventilation means (9) which are controlled by the second control module (82) and are designed to generate a secondary diffusing air jet (D') which is intended to distribute the cold plasma in the vicinity of the drying device (1),
and **in that** the second control module (82) is designed such that the secondary ventilation means (9) are controlled during a treatment cycle in an active diffusing configuration designed to generate a secondary diffusing air jet (D') intended to distribute the cold plasma in the vicinity of the drying device (1).

9. Method of operating a drying device (1) according to one of claims 1 to 8, **characterized in that** the ventilation means (6) are controlled in an active configuration with successive drying cycles,
and **in that** the means (7) for generating plasma are controlled in an active configuration with regular successive treatment cycles independent of the successive drying cycles.

10. Method of operating a drying device (1) according to claim 9, **characterized in that** the successive treatment cycles are regularly carried out at at least one rate, or even at at least two different rates.

11. Method of operating a drying device (1) according to one of claims 9 or 10, **characterized in that** the control of the ventilation means (6) is independent of the successive treatment cycles.

12. Method of operating a drying device (1) according to one of claims 9 or 10, **characterized in that** the control of the ventilation means (6) is correlated with the successive treatment cycles such that the ventilation means (6) are controlled during a treatment cycle and outside a drying cycle at a diffusion velocity which is lower than the drying velocity and is suitable to generate a diffusing air jet (D) which is intended to distribute the cold plasma in the vicinity of the drying device (1).
